# EUROPÄISCHE PATENTSCHRIFT

(11) **EP 1 417 027 B1**
(45) Veröffentlichungstag und Bekanntmachung des Hinweises auf die Patenterteilung: **21.12.2005**
(21) Anmeldenummer: 02735137.8
(22) Anmeldetag: 18.03.2002
(51) Int. Cl.: B01J 19/00, G01N 33/50

(54) **OBERFLÄCHE MIT EINEM MUSTER AUS ZELLEN UND VERFAHREN ZUR HERSTELLUNG DERSELBEN**
SURFACE COMPRISING A PATTERN OF CELLS AND METHOD FOR PRODUCTION THEREOF
SURFACE COMPORTANT UN MOTIF DE CELLULES ET PROCEDE DE FABRICATION DE CETTE SURFACE

(30) Priorität: 19.03.2001 EP 01106789
(43) Veröffentlichungstag der Anmeldung: 12.05.2004
(73) Patentinhaber: Genovac GmbH, 79111 Freiburg (DE)
(72) Erfinder: MAIER, Rainer, 79100 Freiburg (DE); LAUFEN, Thomas, Dr., 81667 München (DE)
(74) Vertreter: Stürken, Joachim
(86) Internationale Anmeldenummer: PCT/EP2002/002968
(87) Internationale Veröffentlichungsnummer: WO 2002/074434

(56) Entgegenhaltungen:
- EP-A- 1 170 591
- WO-A-98/38490
- WO-A-98/54223
- WO-A-99/28744
- WO-A-99/61653
- US-A- 5 955 289
- US-A- 6 103 479

## Beschreibung

Die Erfindung betrifft eine Oberfläche mit einem Muster aus kovalent darauf in Monolayern immobilisierten Zellen, Zellfragmenten, membranösen Zellorganellen oder Membranvesikeln, sowie ein Verfahren zur Herstellung derselben.

Eine Aufstellung der in der folgenden Schilderung des Standes der Technik in Bezug genommenen Literaturstellen mit genaueren bibliographischen Angaben findet sich am Ende dieser Beschreibung.

In den letzten Jahren haben in der Analytik Mikrotechniken immer mehr an Bedeutung gewonnen und es sind zahlreiche Festphasensysteme auf der Grundlage von sich selbst organisierenden Monoschichten (engl. *»self-assembled monolayers«,* »SAMs«) aus bifunktionellen Molekülen (engl. *»Crosslinker«* bzw. »*Linker*«) entwickelt worden, über die spezifisch Probenmoleküle an die Oberfläche des festen Trägers gekoppelt bzw. konjugiert werden, an der dann auch der Nachweis mit Hilfe von geeigneten Markierungen (beispielsweise radioaktiv, gefärbt, fluoreszierend) erfolgt.

Für diese Systeme hat sich in Analogie zu den elektronischen Mikrochips die Bezeichnung Sensorchips eingebürgert. Im Falle der Konjugation von biologischen Molekülen (sog. »Biokonjugation«) an solche Sensorchips, beispielsweise Oligonukleotiden oder Proteinen wie Antikörpern, spricht man auch von Biochips. Die Kopplung an die Trägeroberfläche kann direkt oder indirekt erfolgen. Ein Beispiel für eine indirekte Kopplung ist die Kopplung einer nachzuweisenden Nukleinsäuresequenz durch Hybridisierung an ein immobilisiertes, komplementäres Oligonukleotid als Sonde. In diesem Fall hat die Verwendung der Sonde noch den Vorteil der natürlichen Spezifität der Wechselwirkung biologischer Makromoleküle.

Typischerweise werden zur Herstellung von Sensorchips Oberflächen aus Metall- bzw. Halbmetalloxiden, wie z.B. Aluminiumoxid, Quarzglas, Glas, in eine Lösung von mono- oder bifunktionellen Molekülen bzw. Vernetzern (sog. »Crosslinker« bzw. »Linker«), die beispielsweise eine Halogensilan- (z.B. Chlorsilan-) oder Alkoxysilangruppe zur Kopplung an die Trägeroberfläche aufweisen, getaucht, so daß sich eine sich selbst organisierende Monoschicht (SAM) bildet. Diese weist in diesem Fall eine Dicke von wenigen Ångström auf. Die Kopplung der Linker an die Proben- oder Sondenmoleküle erfolgt über eine geeignete weitere funktionelle Gruppe, beispielsweise eine Amino- oder Epoxygruppe. Geeignete mono-oder bi- funktionelle Linker für die Kopplung einer Vielzahl von Proben- oder Sonden-Molekülen, insbesondere auch biologischen Ursprungs, an eine Vielzahl von Trägeroberflächen sind dem Fachmann gut bekannt, vgl. beispielsweise »Bioconjugate Techniques« von G. T. Hermanson, Academic Press 1996. Weiterhin sind zur Biokonjugation solche Oberflächen geeignet, die aus Polymertägern wie insbesondere funktionalisierten Polymeren bestehen (s. z.B. WO 01/88535).

Diese Technik läßt sich auch auf Zellen übertragen, und es sind derzeit verschiedene Verfahren bekannt, die es ermöglichen Zellen an Oberflächen zu koppeln und beispielsweise immunologisch nachzuweisen.

Von der Firma BIACore, Freiburg, Deutschland ist ein Sensorchip (CM5) erhältlich, der auf seiner Oberfläche Dextranfibrillen trägt, die Carboxymethylgruppen tragen. Durch EDC/NHS-Crosslinking (EDC = 1-Ethyl-3-(3 dimethylaminopropyl) carbodiimid, NHS = N-Hydroxysuccinimidester) können z.B. IgG (1) und Fibronektin (2) an den Chip gekoppelt werden, über die dann Bakterienzellen auf dem Chip immobilisiert werden können. Bei den genannten, zur Kopplung verwendeten Chemikalien handelt es sich beispielsweise um ein Carbodiimid und ein Nukleophil. Besonders geeignet ist z.B. ein Nukleophil, welches das Carbodiimid substituiert und die Carboxygruppe in einen aktivierten Ester überführt, z.B. ein cyclisches Imid wie NHS, S-NHS (= Sulfo-NHS) und HOAt (1-Hydroxyazabenzotriazol). Beide Stoffklassen sind sehr verbreitet zur Kopplung von Carboxy- und Aminogruppen aneinander, d.h. z.B. einer Carboxylmethylgruppe der Dextranfibrillen auf dem BIACore-Sensorchip, an eine Aminogruppe eines Proteins.

Außerdem ist es möglich durch Kopplung von Concanavalin A (1) Erythrozyten auf dem Chip zu immobilisieren. Die so immobilisierten Zellen können dann auf Bindung an Biomoleküle untersucht werden, wobei der observierte Parameter die Änderung des Plasmonresonanzspektrums ist.

Bei einem weiteren Verfahren, ebenfalls von der Firma BIACore, könnten Zellen durch hydrophobe Wechselwirkung mit der Chipoberfläche interagieren. Der HPA-Chip trägt einen sog. »self assembling monolayer« (SAM) an den Liposomen gebunden werden können. Dieses Verfahren wäre auch zur Kopplung von Zellen anwendbar.

Außerdem ist die Kopplung von Zellen an Poly-L-Lysin-Oberflächen sehr weit verbreitet, z.B. auf »Poly-Prep-Slides«, Sigma, P-0425. Die Zellen werden jedoch nicht kovalent an diese Oberfläche gebunden und haften nach eigenen Versuchen schlechter als Zellen, die erfindungsgemäß gekoppelt wurden.

Die US 6103479 A offenbart Verfahren zur Herstellung eines Festphasen-fixierten Zell-Arrays sowie Vorrichtungen, die entsprechend hergestellte Zell-Arrays aufweisen, wobei die Fixierung der Zellen an die feste Phase indirekt mittels Zell-bindender Moleküle erfolgt, die zuvor auf die feste Phase aufgebracht worden sind.

Mit den beschriebenen Verfahren des Standes der Technik ist es aber nicht möglich, verschiedene zelltypen unter Musterbildung, d.h. in Form eines adressierbaren »Arrays«, auf einfache und schnelle Weise direkt als Monolayer auf eine Oberfläche, z.B. einen Chip, aufzubringen und kovalent zu immobilisieren. Mit einer solchen Oberfläche bzw. einem solchen Chip ließe sich eine Probe mit verschiedenen Zelltypen in einem Ansatz untersuchen. Bei den Chips nach dem Stand der Technik haften die Zellen über Wechselwirkungen, die schwächer sind als eine kovalente Bindung.

In diesem Zusammenhang wird darauf hingewiesen, daß sich der zuvor verwendete Begriff "direkt" sowohl auf nackte Oberflächen als auch auf Oberflächen bezieht, die über eine geeignete Kopplungsmatrix verfügen; der Begriff "direkt" schließt somit Anwendungen bzw. entsprechende Vorrichtungen aus, bei denen die Kopplung über Zwischenmoleküle erfolgt, die ihrerseits "direkt" an die Oberflächen gebunden sind.

Der vorliegend verwendete Begriff "Array" bezieht sich auf eine rasterartige Anordnung von Zellen, Zellfragmenten, membranösen Zellorganellen oder Membranvesikeln auf einem Träger, wodurch diskrete, räumlich voneinander getrennte Reaktionsbereiche geschaffen werden, in denen gleiche oder verschiedene Biomoleküle der zuvor beschriebenen Art immobilisiert sind.

Aufgabe der Erfindung ist die Bereitstellung einer derartigen Oberfläche bzw. eines derartigen Chips sowie eines Verfahrens zur Herstellung solcher Vorrichtungen.

Die Erfindung beruht auf der überraschenden Erkenntnis, daß eine derartige Oberfläche schnell und einfach erhältlich ist, indem die Oberflächen oder die Zellen (einschließlich Zellfragmente, membranöse Zellorganellen oder Membranvesikel) mit mindestens einem Vernetzungsmittel (Crosslinker) (im Falle von mehreren Vernetzungsmitteln können diese gleich oder unterschiedlich, mono- oder bifunktional und für den Fall eines bifunktionalen Vernetzers homo- oder heterofunktional sein) aktiviert und anschließend die Zellen (einschließlich Zellfragmente, membranöse Zellorganellen oder Membranvesikel) unter Musterbildung in Form von Monolayern auf die Oberfläche gedruckt und kovalent an dieser immobilisiert werden.

Im folgenden ist der Einfachheit halber auch nur von Zellen die Rede, es sind aber stets auch Zellfragmente, membranöse Zellorganellen, Membranvesikeln oder Membranbestandteile gemeint. Membranbestandteile im Sinne der Erfindung sind beispielsweise Oberflächenproteine, Zucker, Lipide, Glycoproteine, aber auch insbesondere rekombinante Proteine, die in entsprechend transformierten Wirtszellen transient exprimiert werden, wie nachfolgend ausgeführt wird.

Vorteilhaft ist dabei, daß nicht nur auf einfache Weise, nämlich durch Drucken, eine Aufbringung und kovalente Immobilisierung von Zellen in Form von Monolayern auf Oberflächen unter Musterbildung möglich ist, sondern das diese außerdem nicht in einer den spezifischen Nachweis von Oberflächenmolekülen, z.B. Proteinen, behindernden oder sogar unmöglich machenden Weise verändert werden. Beispielsweise bleiben zelluläre Membranproteine aufgrund der milden Immobilisierungsbedingungen unverändert, so daß diese bzw. die Zellen immunologisch nachweisbar bleiben.

Dieses Ergebnis ist überraschend, da eigentlich zu erwarten war, daß beim Drucken die Zellen beispielsweise nicht an die Nadel eines Nadeldruckers (Pinprinters) binden oder sie verkleben, oder die Düsen eines »Nonimpact«-Druckers/kontaktlosen Druckers verstopfen und/oder sich keine Monolayer auf der Oberfläche bilden.

Es ist wichtig, daß die gekoppelten Zellen als Monolayer auf der Oberfläche, beispielsweise des Chips, haften, weil sie so bei weiterer Verarbeitung nicht durch Scherkräfte von der Oberfläche, beispielsweise des Chips, gespült werden, die Teile der Zelloberfläche, über die ein Nachweis erfolgen soll, nicht durch Bildung eines Aggregats blockiert werden und die Anzahl der nachzuweisenden Teile der Oberfläche direkt proportional zur bedeckten Oberfläche, z.B. des Biochips, ist. Nur wenn alle drei Voraussetzungen erfüllt sind, ist es möglich, an den gekoppelten Zellen quantitative Messungen vorzunehmen.

Außerdem ist überraschend, daß die Zellen als Ganzes, d.h. in intakter Form, kovalent gebunden an der Oberfläche, beispielsweise des Chips, haften. Es hätte auch geschehen können, daß die Teile der Zelloberfläche, über die die Zellen an die Oberfläche binden, beispielsweise die Oberfläche des Chips, herausgerissen werden.

Die Erfindung betrifft ferner ein Verfahren zum Nachweis von Membranbestandteilen wie Oberflächenproteinen, Zuckern, Sterolen und Lipiden auf Zellen (oder Zellfragmenten, membranösen Zellorganellen oder Membranvesikeln), bei dem ein(e) erfindungsgemäße(r) Oberfläche/Chip mit einem oder mehreren, mit den jeweils nachzuweisenden Membranbestandteilen, z.B. Oberflächenproteinen, spezifisch reagierenden Nachweisreagenzien in Kontakt gebracht wird, ein medizinisches oder diagnostisches Gerät, das eine(n) erfindungsgemäße(n) Oberfläche/Chip aufweist sowie einen Kit zur Verwendung mit einem solchen Gerät, der eine(n) erfindungsgemäße(n) Oberfläche/Chip und entsprechende Nachweisreagenzien enthält.

Neben qualitativen oder quantitativen Messungen für beispielsweise diagnostische Zwecke gestatten die erfindungsgemäßen Oberflächen oder Chips auch strukturelle und funktionelle Untersuchungen an Membranen, da auf einfache Weise durch Wahl geeigneter Crosslinker eine Orientierung der Membran auf der Oberfläche möglich ist, so daß beispielsweise die Verteilung von Oberflächenmolekülen auf der Außenseite oder Innenseite (d.h. zum Zellinneren hin) der Membran bestimmt werden kann oder Untersuchungen an Transmembranproteinen oder Rezeptoren und deren Wechselwirkungen mit Liganden möglich sind.

Es ist ferner klar, daß mit den erfindungsgemäßen Oberflächen/Chips nicht nur die Membranmoleküle der daran immobilisierten Zellen untersucht werden können, sondern auch deren Inhalt, beispielsweise das Cytosol, der Kern oder Zellorganellen bzw. deren Inhalt. Eine bevorzugte Ausführungsform der Erfindung betrifft daher Oberflächen mit im wesentlichen vollständigen Zellen, in denen die interessierenden Proteine (oder andere Strukturen) innerhalb der Zellen, so z.B. im Cytosol, exprimiert werden bzw. worden sind, wobei das Zellinnere für Antikörperbindungen oder andere Rezeptor/Liganden-gestützte Nachweisreaktionen z.B. durch Perforation der Zellmembran (vorübergehend) zugänglich gemacht wird bzw. worden ist. Besonders bevorzugt sind Oberflächen mit einem Muster von Zellen (Zellarray) des selben Zelltyps, wobei sich die Zellen eines jeweiligen Rasterpunktes oder Reaktionsbereiches von denjenigen eines anderen Punktes oder Bereiches lediglich durch die spezifische Expression eines oder mehrerer Proteine auf der Oberfläche unterscheiden.

Auf diese Weise kann die gewünschte Immobilisierung bestimmter Zellen unter vergleichbaren und reproduzierbaren Bedingungen stattfinden, wobei die exprimierten Proteine in weitgehend nativer Form auf der Oberfläche dieser Zellen präsentiert werden. Dies ist insbesondere für die Testung von Antikörpern unter physiologischen Bedingungen von Interesse. Eine besonders bevorzugte Ausführungsform betrifft daher eine erfindungsgemäße Oberfläche, bei der die darauf immobilisierten Zellen, Zellfragmente, membranösen Zellorganellen oder Membranvesikeln rekombinante Proteine umfassen, welche zuvor in entsprechend transformierten Wirtszellen durch transiente Expression exprimiert worden sind. Ein derartiges Verfahren zur Herstellung von Antikörpern ist im deutschen Patent 19852800 beschrieben und kann zur Schaffung der erfindungsgemäßen Oberflächen angewendet werden. Auch können durch Mehrfachtransfektion Proteine bzw. Proteinkomplexe an die Zelloberfläche gebracht werden, die für eine korrekte Einbindung in die Zellmembran Kofaktoren benötigen. Mit derartig beschaffenen Oberflächen können sehr schnell große Mengen an Zellen zur weiteren Analyse bereitgestellt werden. Insbesondere ermöglicht dieser methodische Ansatz die rasterartige Immobilisierung und Analyse von G-Protein gekoppelten Rezeptorproteinen (GPCR), weshalb insbesondere solche Oberflächen bevorzugt sind, bei denen die Proteine GPCR-Rezeptoren sind. Normalerweise lassen sich derartige Rezeptoren aufgrund ihrer Hydrophilie und ihrer Tertiärstruktur nur in Form von Membranvesikeln drucken und immobilisieren. Werden diese Proteine jedoch in entsprechend transformierten Zellen transient exprimiert, so läßt sich mit diesen Zellen in einfacher Weise ein GPCR-Array herstellen. Überaschenderweise stellte sich im Rahmen der zur Erfindung durchgeführten Arbeiten heraus, dass die GPCRs auf den Zellen trotz der Immobilisierung immer noch ihr typisches (natives) Ligandenbindungsverhalten aufweisen, so dass derartige, erfindungsgemäß bereitgestellte Oberflächen zur Entwicklung und Studie von Rezeptor-Agonisten und -Antagonisten geeignet sind. Insbesondere können damit auch die Effekte potentieller Wirkstoffe auf andere Rezeptoren als die eigentlichen Zielrezeptoren untersucht werden. Von vielen pharmakologisch wirksamen Substanzen weiß man heute, dass außer dem Zielrezeptor auch noch andere Rezeptoren in einer durchaus unerwünschten Weise beeinflusst werden. Solche unerwünschten Wirkungen (Nebenwirkungen) führen bei der Verwendung von vieler Antihistaminika z.B. zu Müdigkeitserscheinungen, können aber auch noch wesentlich bedrohlichere Effekte evozieren. Ein weiteres Anwendungsfeld für die erfindungsgemäßen Oberflächen ist die Analyse sogenannter "orphan drugs"; mit diesem Begriff werden Substanzen bezeichnet, deren pharmakologischer Rezeptor bisher unbekannt ist. Seit der erfolgreichen Sequenzierung des humanen Genoms durch das HUGO-Projekt sind alle potentiellen DNA-Sequenzen von GPCRs des menschlichen Genoms verfügbar und können somit in einer GPCR-Expressionsbiliothek zusammen mit den bekannten und charakterisierten GPCSs auf Chips gedruckt und - gegebenenfalls vergleichend - analysiert werden.

Weitere vorteilhafte und/oder bevorzugte Ausführungsformen der Erfindung sind Gegenstand der Unteransprüche.

Die erfindungsgemäß geeigneten Oberflächen unterliegen keinen besonderen Beschränkungen hinsichtlich der chemischen Beschaffenheit, der Form oder der Abmessungen, sofern sie zum Bedrucken geeignet sind.

Beispielsweise können an der Oberfläche freie Epoxy-, Carboxy-, Amino- Aldehyd-, Cyano, Isothiocyano-, Thiocyano-oder Thiolgruppen vorhanden sein, an die ein bifunktioneller Vernetzer zur Aktivierung (und zur späteren kovalenten Immobilisierung der Zellen) kovalent gebunden werden kann oder an die mit einem bifunktionellen Vernetzer entsprechend aktivierte Zellen kovalent immobilisiert werden können. Diese Gruppen können auf der Oberfläche schon von vornherein vorhanden sein wie zum Beispiel auf verschiedenen Membranen oder Polymeren oder durch eine entsprechende Beschichtung aufgebracht werden wie zum Beispiel durch Silanisierung, Bedampfung oder Pfropfung bzw. »Grafting« von Polymer- oder Glaschips, Membranen oder Metalloberflächen. Es können natürlich nicht nur feste Oberflächen verwendet werden, sondern auch »halbfeste«, beispielsweise gelartige Oberflächen, sofern darauf das gedruckte Zellmuster nicht verläuft oder darin absorbiert wird.

Die Vernetzer (Crosslinker) unterliegen ebenfalls keinen besonderen Beschränkungen, und dem Fachmann sind geeignete Vernetzer (Crosslinker) zur Kopplung einer Vielzahl von Biomolekülen an eine Vielzahl von Trägeroberflächen gut bekannt, vgl. beispielsweise »Bioconjugate Techniques« von G. T. Hermanson, Academic Press 1996. Zur Kopplung können ein oder mehrere homo- oder bifunktionale Vernetzer und für den Fall eines binfunktionalen Vernetzers Homo- oder Heterofunktionale verwendet werden.

Im Falle von Oberflächen mit freien Epoxy-, Carboxy- oder Aminogruppen eignen sich als Vernetzer (Crosslinker) beispielsweise ein Carbodiimid und allgemein ein Nukleophil, beispielsweise ein Nukleophil, welches das Carbodiimid substituiert und die Carboxygruppe in einen aktivierten Ester überführt, z.B. ein cyclisches Imid wie NHS (= N-Hydroxysuccinimidester), S-NHS (= Sulfo-NHS) und HOAt (= 1-Hydroxyazabenzotriazol).

Als Vernetzer (Crosslinker) eignen sich hinsichtlich des Carbodiimids beispielsweise EDC (= 1-Ethyl-3-(3dimethylaminopropyl)carbodiimid oder DICD (= Diisopropyl-carbodiimid) und hinsichtlich des Nucleophils beispielsweise NHS, S-NHS oder HOAt.

Das Carbodiimid, beispielsweise EDC oder DICD, und das Nukleophil, beispielsweise NHS oder S-NHS, können grundsätzlich unabhängig voneinander verwendet werden, d.h. es ist keine Kombination erforderlich. Die Kombination von beispielsweise EDS/S-NHS wird aber hier bevorzugt, weil sich eine wesentlich bessere Immobilisierung als bei der Verwendung nur einer der beiden Komponenten ergibt und S-NHS (gegenüber NHS) den Vorteil hat, das es wasserlöslich ist und sich sonst keine weitere Eigenschaft ändert.

Es ist ferner klar, daß es dem Fachmann freisteht, ob er die Oberfläche und/oder die Zellen mit einem geeigneten Vernetzer (Crosslinker) aktiviert. Im Falle der Aktivierung der Oberfläche kann sich der Aktivierung ein Waschschritt zur Entfernung von überschüssigem Vernetzungsmittel anschließen. Falls die Zellen aktiviert werden, wird die Konzentration des Vernetzungsmittels zweckmäßigerweise so gewählt, daß die Zellen nicht miteinander vernetzen. Geeignete Konzentrationsbereiche lassen sich mit einfachen Versuchen bestimmen.

Eine weitere erfindungsgemäß geeignete Möglichkeit zur Immobilisierung von Zellen auf Oberflächen besteht in der Verwendung von photoreaktiven Crosslinkern. Beispielsweise lässt sich Glas mit einem Benzophenonsilan beschichten und dann mit Zellen bedrucken. Eine anschließende kurze UV-Bestrahlung bei 300 nm erzeugt aus dem Benzophenon Radikale, mit deren Hilfe beliebige Zellbestandteile kovalent gebunden werden können.

Für zahlreiche analytisch/diagnostische Anwendungen kann es gewünscht sein, dass die Zellen, Zellfragmente, membranösen Zellorganellen oder Membranvesikeln fixiert werden, wobei die Fixierung unter Verwendung herkömmlicher Agenzien (z.B. Glutaraldehyd) vor oder nach dem Drucken erfolgen kann. Insbesondere in Fällen, bei denen eine bedruckte Oberfläche gelagert werden soll, kann eine Fixierung von Vorteil sein, damit die nativen Eigenschaften von in den Zellen, Zellfragmenten, membranösen Zellorganellen oder Membranvesikeln vorliegenden Biomolekülen (Proteine, Rezeptoren etc.) für später durchzuführende Studien und Analysen erhalten bleiben. Beispielsweise wird auf die native Eigenschaft der spezifischen Bindung eines zu analysierenden Oberflächenrezeptors verwiesen.

Zum Drucken kann beispielsweise ein Nadeldrucker oder ein »Nonimpact«-Drucker/kontaktloser Drucker verwendet werden. Da Nadeldrucker weitverbreitet und besonders unanfällig gegen Störungen sind (es können keine Düsen verstopfen) werden diese hier bevorzugt verwendet. Zum Drucken von Mustern (»patterned arrays«) eignet sich z.B. eine Nadel mit einem Spot-Durchmesser 200 µm. Außerdem können Nadeln verwendet werden, die Spots mit anderen Durchmessern drucken. Eine untere Grenze liegt derzeit bei ca. 100 µm, eine obere Grenze wurde auch mit 600 µm nicht erreicht.

Hinsichtlich der immobilisierbaren Zellen bestehen ebenfalls keine besonderen Beschränkungen, es können beliebige Zellen prokaryotischen oder eukaryotischen Ursprungs verwendet werden. Ebenfalls verwendet werden können Zellfragmente oder Zelltrümmer, Membranvesikel (z.B. »Inverted Vesicels«, ER-Vesikel, membranöse Organellen (Mitochondrien, Plastiden, Vakuolen etc.) oder Fragmente oder Trümmer davon.

Die Nachweisreagenzien unterliegen keinen besonderen Beschränkungen und können einen oder mehrere unmarkierte oder markierte polyklonale oder monoklonale Antikörper, chimäre Antikörper oder »Single-chain«-Antikörper oder funktionelle Fragmente oder Derivate davon umfassen. Außerdem können im Falle von spezifisch Liganden bindenden Oberflächenstrukturen (beispielsweise Rezeptoren) diese Liganden (ggf. nach entsprechender Markierung) verwendet werden, z.B. entsprechende Hormone für Hormonrezeptoren, entsprechende Biomoleküle für ABC-Transporter, selektive Poren oder Kanäle.

Die Markierung kann beliebig und beispielsweise ausgewählt sein unter radioaktiven, farbigen, fluoreszierenden, biolumineszierenden, chemilumineszierenden oder phosphoreszierenden Markierungen, oder auf einem Enzym, einem Antikörper oder einem funktionellen Fragment oder Derivat davon, oder auf einem Protein-A/Gold-, Protein-G/Gold oder Avidin/ Streptavidin/Biotin-System beruhen.

Es ist klar, daß die eigentliche Nachweisreaktion direkt oder indirekt erfolgen kann. Im Falle von beispielsweise Antikörpern als Nachweisreagenz könnte der direkte Nachweis auf der Bindung von markierten spezifischen Antikörpern beruhen. Ein indirekter Nachweis könnte auf der Bindung eines unmarkierten primären Antikörpers an ein Antigen (z.B. ein Oberflächenprotein) und der sich anschließenden Bindung eines markierten sekundären Antikörpers gegen den primären Antikörper (sog. »Anti-Antikörper«) beruhen (sog. »Sandwich«-Verfahren).

Im folgenden wird die Erfindung ohne Beschränkung detaillierter beschrieben.

Die Immobilisierung beliebiger Zellen, Zellfragmente, membranöser Zellorganellen oder Membranvesikel kann beispielsweise folgendermaßen erfolgen.

Zum Fixieren der Zellen werden ca. 8•10⁶ Zellen in 1 ml 150 mM PBS, pH 7.2 aufgenommen. Unter langsamen Vortexen wird 10% Formaldehyd, 10% Paraformaldehyd oder 10% Glutaraldehyd zu einer Endkonzentration von 0.5% (v/v) zugegeben. Die Zellen werden 30 min auf Eis inkubiert, alle 10 min kurz »gevortext« und schließlich bei max. 400g 5 min bei 4°C pelletiert. Anschließend werden die Zellen 2mal in 1 ml 150 mM PBS pH 7.2 gewaschen. Die so behandelten Zellen sind bei 4°C bis zu zwei Wochen lagerfähig. Auf den Fixierungsschritt kann verzichtet werden, die Zellen verlieren dann allerdings nach kurzer zeit (wenige Tage) ihre Immunogenität.

Zum Drucken werden 8•10⁶ Zellen mit max. 400 x g bei 4°C 5 min pelletiert, so daß ein Pellet mit ca. 50 µl Volumen entsteht. Das Pellet wird in 200 µl 150 mM PBS pH 7.2 aufgenommen, das zusätzlich 6.3 mM EDC und 0.92 mM S-NHS zum Vernetzen (»crosslinking«) enthält und 5 min bei Raumtemperatur inkubiert. Als Vernetzer können, wie oben beschrieben, auch andere Carbodiimide oder Nukleophile verwendet werden. Es können auch andere Verhältnisse von EDC und S-NHS verwendet werden. Es ist auch möglich, auf eine der beiden Komponenten, das Carbodiimid oder das Nukleophil, zu verzichten. Allerdings ergibt sich bei kombinierter Anwendung eine wesentlich bessere Immobilisierung. Nach der Inkubation werden die Zellen erneut pelletiert und der Überstand verworfen. Das so gewonnene Pellet kann direkt zum Drucken verwendet werden.

Zum Drucken wird das Pellet in ELISA Platten überführt. 384-Well-Platten (»*well*« = *engl. »Vertiefungen«*) werden mit 40 µl Pellet pro Well befüllt, 96-Well-ELISA-Platten werden mit 40 µl Pellet pro Well befüllt. Unter Verwendung eines Array-Druckers Modell Omnigrid, GeneMachines, und einer Nadel mit 200 µm Spot-Durchmesser werden die Zellen in einem Array aufgedruckt, wobei eine Nadel nach der Beladung ca. 10 Spots absetzen kann.

Zum Koppeln der Zellen und zum Blocken der Chipoberfläche werden die bedruckten Chips unmittelbar nach dem Druck zunächst zwei Stunden bei Raumtemperatur inkubiert, um die Zellen kovalent an den Chip zu koppeln. Dabei können die Zellen trocken werden oder feucht bleiben. Anschließend werden die Chips 30 min in 100 mM Boratpuffer bei pH 9.5 inkubiert, um verbliebene aktivierte Ester zu hydrolysieren. Die Hydrolyse kann auch mit einem anderen anorganischen Puffer bei diesem pH durchgeführt werden.

Danach werden die Zellen 2mal 5 min in 150 mM PBS pH 7.2 gewaschen. Zum Blocken der Oberfläche werden die Chips dann 1 h in 3% BSA in 150 mM PBS bei pH 7.2 inkubiert und danach 2mal 5 min in Cova-Puffer (2 M NaCl, 10 % MgSO₄, 150 mM PBS pH 7.2, 0,1 % Tween 20) gewaschen.

Die Zelloberflächenproteine werden durch einen Erstantikörper (Primärantikörper) nachgewiesen (im hier beschriebenen Fall mit einem monoklonalen α-myc-Antikörper, 0.003 mg/ml, 1 h Inkubationszeit bei Raumtemperatur). Der Chip wird erneut 2mal 5 min in Cova-Puffer gewaschen, worauf der Erstantikörper durch einen markierten Zweitantikörper (Sekundärantikörper oder Anti-Antikörper) nachgewiesen wird. (Im hier beschriebenen Fall Esel-α-Maus-Cy5, 0.005 mg/ml, 1 h Inkubationszeit bei Raumtemperatur). Der Chip wird erneut 2mal 5 min in Cova-Puffer gewaschen, vorsichtig mit Stickstoff trockengeblasen und ausgelesen.

Die besten Ergebnisse wurden mit fixierten (wie oben beschrieben) und mit mittels EDC/S-NHS kovalent auf dem Chip immobilisierten Zellen erzielt.

### Bibliographie

1. BIAtechnology Note 103, BIAcore AB
2. Holmes, S. et al. (1994), präsentiert auf dem 4. European BIAsymposium, Heidelberg

## Patentansprüche

1. Oberfläche mit einem darauf befindlichen Muster aus Zellen, Zellfragmenten, membranösen Zellorganellen oder Membranvesikeln, welche auf der Oberfläche in Form von Monolayern mittels Vernetzer direkt kovalent immobilisiert sind.

2. Oberfläche mit einem Muster aus kovalent darauf in Monolayern immobilisierten Zellen, Zellfragmenten, membranösen Zellorganellen oder Membranvesikeln, erhältlich nach einem Verfahren, bei dem
a) eine Oberfläche und/oder Zellen, Zellfragmente, membranöse Zellorganellen oder Membranvesikeln mit einem Vernetzer aktiviert wird/werden, und anschließend
b) die Zellen, Zellfragmente, membranösen Zellorganellen oder Membranvesikeln unter Musterbildung in Form von Monolayern direkt auf die Oberfläche gedruckt und kovalent an dieser immobilisiert werden.

3. Verfahren zur Herstellung einer Oberfläche mit einem Muster aus kovalent darauf in Monolayern immobilisierten Zellen, Zellfragmenten, membranösen Zellorganellen oder Membranvesikeln nach Anspruch 1 oder 2, bei dem
a) eine Oberfläche und/oder Zellen, Zellfragmente, membranöse Zellorganellen oder Membranvesikeln mit mindestens einem bifunktionellen Vernetzer aktiviert wird/werden, und anschließend
b) die Zellen, Zellfragmente, membranösen Zellorganellen oder Membranvesikeln unter Musterbildung in Form von Monolayern auf die Oberfläche gedruckt und kovalent an dieser immobilisiert werden.

4. Verfahren nach Anspruch 3, bei dem die Zellen, Zellfragmente, membranösen Zellorganellen oder Membranvesikeln vor oder nach dem Drucken chemisch fixiert werden.

5. Oberfläche, erhalten nach einem Verfahren gemäß Anspruch 3 oder 4.

6. Oberfläche oder Verfahren nach einem der Ansprüche 2 bis 5, wobei die Oberfläche von Schritt a) des Anspruches 2 oder 3 Amino-, Carboxy- oder Epoxygruppen aufweist und als Vernetzer ein Carbodiimid und ein Nukleophil verwendet werden.

7. Oberfläche oder Verfahren nach Anspruch 6, wobei das Carbodiimid 1-Ethyl-3-(3dimethylaminopropyl)carbodiimid oder Diisopropylcarbodiimid und das Nukleophil Sulfo-N-Hydroxysuccinimidester ist.

8. Oberfläche oder Verfahren nach einem der Ansprüche 2 bis 5, wobei zur Immobilisierung ein UV-induzierbarer Vernetzer verwendet wird.

9. Oberfläche oder Verfahren nach einem der vorstehenden Ansprüche, wobei die Zellen prokaryotischen oder eukaryotischen Ursprungs sind.

10. Oberfläche oder Verfahren nach einem der vorstehenden Ansprüche, wobei die Zellen, Zellfragmente, membranösen Zellorganellen oder Membranvesikeln rekombinante Proteine umfassen, welche zuvor durch transiente Expression gebildet worden sind.

11. Oberfläche oder Verfahren nach Anspruch 10, bei dem die Proteine GPCR-Rezeptoren sind.

12. Oberfläche oder Verfahren nach einem der Ansprüche 2 bis 8, wobei zum Drucken ein Nadeldrucker verwendet wird.

13. Oberfläche nach einem der Ansprüche 1, 2, und 5 bis 12, die Teil eines Sensorchips oder Biochips ist.

14. Verfahren zum Nachweis von Oberflächenmolekülen auf Zellen, Zellfragmenten, membranösen Zellorganellen oder Membranvesikeln, oder von Molekülen im Inneren der Zellen, bei dem eine Oberfläche mit einem direkt darauf befindlichen Muster aus Zellen, Zellfragmenten, membranösen Zellorganellen oder Membranvesikeln, welche auf der Oberfläche in Form von Monolayern mittels Vernetzer kovalent immobilisiert sind, mit einem oder mehreren, mit den jeweils nachzuweisenden Oberflächenmolekülen oder Molekülen aus dem Inneren der Zellen spezifisch reagierenden Nachweisreagenzien in Kontakt gebracht wird.

15. Verfahren nach Anspruch 14, wobei die Nachweisreagenzien einen oder mehrere unmarkierte oder markierte polyklonale oder monoklonale Antikörper, chimäre Antikörper oder »Single-chain«-Antikörper oder funktionelle Fragmente oder Derivate davon umfassen.

16. Verfahren nach Anspruch 15, wobei die Markierung ausgewählt ist unter radioaktiven, farbigen, fluoreszierenden, biolumineszierenden, chemilumineszierenden oder phosphoreszierenden Markierungen, oder auf einem Enzym, einem Antikörper oder einem funktionellen Fragment oder Derivat davon, oder auf einem Protein-A/Gold-, Protein-G/Gold- oder Avidin/Streptavidin/Biotin-System beruht.

17. Medizinisches oder diagnostisches Gerät, das eine Oberfläche nach einem der Ansprüche 1, 2, und 5 bis 13 aufweist.

18. Kit zur Verwendung mit einem Gerät nach Anspruch 17, umfassend eine Oberfläche nach einem der Ansprüche 1, 2, und 5 bis 13, und ein oder mehrere Nachweisreagenzien ausgewählt aus der Gruppe bestehend aus einem oder mehreren unmarkierten oder markierten polyklonalen oder monoklonalen Antikörpern, chimären Antikörpern oder »Single-chain«-Antikörpern, und funktionellen Fragmenten oder Derivaten davon.

19. Kit nach Anspruch 18, umfassend eine Oberfläche nach Anspruch 10 oder 11.

20. Verwendung des Kits nach Anspruch 18 oder 19 zur Analyse des Bindungsverhaltens zwischen Rezeptoren und Liganden.

## Claims

1. A surface with a pattern of cells, cell fragments, membranous cell organelles or membrane vesicles, which are directly covalently immobilized on the surface in the form of monolayers by crosslinkers.

2. A surface with a pattern of cells, cell fragments, membranous cell organelles or membrane vesicles, which are covalently immobilized thereon as monolayers, obtainable by a method, wherein
a) a surface and/or cells, cell fragments, membranous cell organelles or membrane vesicles is/are activated with a crosslinker, and subsequently
b) the cells, cell fragments, membranous cell organelles or membrane vesicles are printed directly onto the surface in the form of monolayers and are covalently immobilized thereon, thereby creating a pattern.

3. A method for the production of a surface with a pattern of cells, cell fragments, membranous cell organelles or membrane vesicles, which are covalently immobilized thereon as monolayers, according to claim 1 or 2, wherein
a) a surface and/or cells, cell fragments, membranous cell organelles or membrane vesicles is/are activated with at least one bifunctional crosslinker, and subsequently
b) the cells, cell fragments, membranous cell organelles or membrane vesicles are printed directly onto the surface in the form of monolayers and are covalently immobilized thereon, thereby creating a pattern.

4. A method according to claim 3, wherein the cells, cell fragments, membranous cell organelles or membrane vesicles are chemically fixed before or after printing.

5. A surface, obtained by a method as defined in claim 3 or 4.

6. A surface or a method according to any of claims 2 to 5, wherein the surface from step a) of claim 2 or 3 has amino, carboxy, or epoxy groups, and wherein a carbodiimide and a nucleophile are used as crosslinker.

7. A surface or a method according to claim 6, wherein the carboiimide is 1-ethyl-3-(3-dimethylaminopropyl)carbodiimide or diisopropylcarbodiimide, and wherein the nucleophile is sulfo-N-hydroxysuccinimide ester.

8. A surface or a method according to any of claims 2 to 5, wherein an UV-inducible crosslinker is used for immobilization.

9. A surface or a method according to any of the preceding claims, wherein the cells are of procaryotic or eucaryotic origin.

10. A surface or a method according to any of the preceding claims, wherein the cells, cell fragments, membranous cell organelles or membrane vesicles comprise recombinant proteins, which had been generated before by transient expression.

11. A surface or a method according to claim 10, wherein the proteins are GPCR-receptors.

12. A surface or a method according to any of claims 2 to 8, wherein a matrix printer is used for printing.

13. A surface according to any of claims 1, 2, and 5 to 12, which is part of a sensor chip or biochip.

14. A method for the detection of surface molecules on cells, cell fragments, membranous cell organelles or membrane vesicles, or of molecules within said cells, wherein a surface having directly thereon a pattern of cells, cell fragments, membranous cell organelles or membrane vesicles, which are covalently immobilized on said surface in the form of monolayers by crosslinkers, is brought into contact with one or more detection reagents specifically reacting with the surface molecules or molecules from within the cells to be detected.

15. A method according to claim 14, wherein the detection reagents comprise one or more unlabelled or labelled polyclonal or monoclonal antibodies, chimeric antibodies or single-chain antibodies or functional fragments or derivatives thereof.

16. A method according to claim 15, wherein the label is selected from radioactive, coloured, fluorescent, bioluminescent, chemoluminescent or phosphorescent labels, or is based on an enzyme, an antibody or a functional fragment or derivative thereof, or on a protein-A/gold, protein-G/gold, or avidine/streptavidine/biotin system.

17. A medical or diagnostic device, which has a surface as defined in any of claims 1, 2, and 5 to 13.

18. Kit for use with a device as defined in claim 17, comprising a surface as defined in any of claims 1, 2, and 5 to 13, and one or more detection reagents selected from the group consisting of one or more unlabelled or labelled polyclonal or monoclonal antibodies, chimeric antibodies or single-chain antibodies, and functional fragments or derivatives thereof.

19. Kit according to claim 18, comprising a surface as defined in claim 10 or 11.

20. Use of the kit as defined in claim 18 or 19 for the analysis of the binding behaviour between receptors and ligands.

## Revendications

1. Une surface sur laquelle se trouve un pattern composé de cellules, fragments de cellules, organelles cellulaires comportant des membranes ou vésicules membranaires, qui sont directement immobilisés de manière covalente sous forme de monocouche sur la surface à l'aide de liant.

2. Une surface avec un pattern de cellules, fragments de cellules, organelles cellulaires comportant des membranes ou vésicules de membrane, immobilisés de manière covalente en monocouche obtenue par un procédé **caractérisé en ce que** :
a) une surface et/ou des cellules, fragments de cellules, organelles cellulaires comportant des membranes ou vésicules membranaires est/sont activé(s) par un liant, et ensuite
b) les cellules, les fragments de cellules, les organelles cellulaires comportant des membranes ou les vésicules membranaires sont directement imprimés sur la surface sous forme de monocouche selon un pattern et sont immobilisés de manière covalente sur celle-ci.

3. Un procédé pour la production d'une surface avec un pattern de cellules, fragments de cellules, organelles cellulaires comportant des membranes ou vésicules membranaires immobilisés de manière covalente en monocouche sur celle-ci, selon les revendications 1 et 2, **caractérisé en ce que**
a) une surface et/ou des cellules, des fragments de cellules, des organelles cellulaires comportant des membranes ou des vésicules membranaires est/sont activé(s) avec au moins un liant bifonctionnel, et ensuite
b) les cellules, les fragments de cellules, les organelles cellulaires comportant des membranes ou les vésicules membranaires sont imprimés selon un pattern sous forme de monocouche sur la surface et sont immobilisés de manière covalente sur celle-ci.

4. Un procédé selon la revendication 3, **caractérisé en ce que** les cellules, les fragments de cellules, les organelles cellulaires comportant des membranes ou les vésicules membranaires sont fixés chimiquement avant ou après l'impression.

5. Une surface obtenue par un procédé selon la revendication 3 ou 4.

6. Une surface ou un procédé selon une des revendications 2 à 5, **caractérisé en ce que** la surface de l'étape a) des revendications 2 ou 3 possède des groupes amino-, carboxy-ou epoxy et où on utilise comme liant un carbodiimide et un nucléophile.

7. Une surface ou un procédé selon la revendication 6, où le carbodiimide est le carbodiimide 1-éthyl-3-(3 diméthylaminopropyle) ou le carbodiimide diisopropyle, et le nucléophile est l'ester sulfo-N d'hydroxysuccinimide.

8. Une surface ou un procédé selon une des revendications 2 à 5, où on utilise pour l'immobilisation un liant inductible par UV.

9. Une surface ou un procédé selon une des revendications précédentes, où les cellules sont d'origine procaryote ou eucaryote.

10. Une surface ou un procédé selon une des revendications précédentes, où les cellules, les fragments de cellules, les organelles cellulaires comportant des membranes ou les vésicules membranaires contiennent des protéines recombinantes, lesquelles ont été produites à l'avance par expression transitoire.

11. Une surface ou un procédé selon la revendication 10, où les protéines sont des récepteurs GPCR.

12. Une surface ou un procédé selon une des revendications 2 à 8, où on utilise pour l'impression une imprimante à aiguille.

13. Une surface selon une des revendications 1, 2 et 5 à 12 qui fait partie d'une puce sensorielle ou une puce biologique.

14. Un procédé pour la détection de molécules de surface de cellules, de fragments de cellule, d'organelles cellulaires comportant des membranes ou des vésicules membranaires, ou de molécules internes aux dites cellules, **caractérisé en ce que** une surface sur laquelle se trouve directement un pattern de cellules, de fragments de cellules, d'organelles cellulaires comportant des membranes ou de vésicules membranaires, immobilisés de manière covalente par un liant sous forme de monocouche, est mise en contact avec un ou plusieurs réactifs de détection réagissant spécifiquement avec respectivement les dites molécules de surface ou les dites molécules internes des cellules à détecter.

15. Un procédé selon la revendication 14 **caractérisé en ce que** les réactifs de détection incluent un ou plusieurs anticorps polyclonaux ou monoclonaux, des anticorps chimériques ou des anticorps à une seule chaîne ou des fragments ou des dérivés fonctionnels de ceux-ci, marqués ou non.

16. Un procédé selon la revendication 15 **caractérisé en ce que** le marquage est choisi parmi les marquages radioactifs, colorés, fluorescents, bioluminescents, chemiluminescents ou phosphorescents, ou est basé sur un enzyme, un anticorps ou un fragment ou dérivé fonctionnel de celui-ci, ou sur un système protéine-A/or, protéine-G/or ou le système avidine/streptavidine/biotine.

17. Un appareil à but médical ou diagnostique qui comprend une surface selon une des revendications 1,2 et 5 à 13.

18. Un kit utilisé avec un appareil selon la revendication 17, incluant une surface selon une des revendications 1,2, et 5 à 13, et un ou plusieurs réactifs de détection choisis dans le groupe comprenant un ou plusieurs anticorps polyclonaux ou monoclonaux, des anticorps chimériques ou des anticorps à une seule chaîne, ou des fragments ou dérivés fonctionnels de ceux-ci, marqués ou non.

19. Un kit selon la revendication 18 incluant une surface selon les revendication 10 ou 11.

20. Utilisation d'un kit selon les revendications 18 ou 19 pour l'analyse du mode de liaison entre récepteurs et ligands.
